# EUROPÄISCHE PATENTSCHRIFT

(11) **EP 4 214 266 B1**
(45) Veröffentlichungstag und Bekanntmachung des Hinweises auf die Patenterteilung: **09.12.2026**
(21) Anmeldenummer: 21773809.5
(22) Anmeldetag: 14.09.2021
(51) Int. Cl.: C08G 69/46, C08G 69/14, C07C 227/22, C07D 225/02, C08J 11/14

(54) **VERFAHREN ZUR SAUREN HYDROLYSE VON REINEM POLYLAURINLACTAM**
PROCESS FOR ACIDIC HYDROLYSIS OF PURE POLYLAUROLACTAM
PROCÉDÉ D'HYDROLYSE ACIDE DE POLYLACTAME LAURIQUE PURE

(30) Priorität: 16.09.2020 EP 20196421
(43) Veröffentlichungstag der Anmeldung: 26.07.2023
(73) Patentinhaber: Evonik Operations GmbH, 45128 Essen (DE)
(72) Erfinder: RICHTER, Alexander, 45721 Haltern am See (DE); NÖRNBERG, Christian, 22045 Hamburg (DE); KERN, Norbert, 45721 Haltern (DE); HERMES, Florian, 45721 Haltern am See (DE); BAUMANN, Franz-Erich, 48249 Dülmen (DE); ROOS, Martin, 45721 Haltern am See (DE); DEMICOLI, Daniel, 45133 Essen (DE)
(74) Vertreter: Evonik Patent Association
(86) Internationale Anmeldenummer: PCT/EP2021/075148
(87) Internationale Veröffentlichungsnummer: WO 2022/058291

(56) Entgegenhaltungen:
- US-A- 4 170 588
- US-A1- 2019 016 668

## Beschreibung

Die vorliegende Erfindung betrifft ein verbessertes Verfahren zur sauren Hydrolyse von Polylaurinlactam mit Schwefelsäure. Als Ausgangsmaterial eignet sich besonders Polylaurinlactam, was wiederverwertet werden soll und sich durch einen geringen Gehalt an Laurinlactam auszeichnet.

### Hintergrund der Erfindung

Polylaurinlactam (Cas-Nr.: 24937-16-4; alternative Bezeichnungen: Nylon 12, Polyamid 12; im Folgenden abgekürzt mit "PA12") ist ein industriell wichtiges Polymer.

PA12 umfasst Wiederholungseinheiten der folgenden Struktur **(I),** wobei die mit (*) bezeichnete Bindung einer Wiederholungseinheit an die mit (**) der benachbarten Wiederholungseinheit bindet.

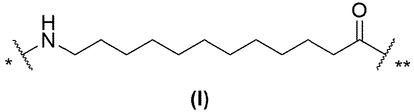

PA12 wird in Anwendungen u. a. für seine hohe Hydrolysebeständigkeit geschätzt und differenziert sich durch diese Eigenschaft klar von kurzkettigen Polyamiden wie Polycaprolactam
(Cas-Nr.: 25038-54-4; alternative Bezeichnung: Polyamid 6; im Folgenden abgekürzt mit "PA6") oder Poly-(*N*,*N'*-hexamethylenadipindiamid)/ Poly-(hexamethylenadipamid) (Cas-Nr.: 32131-17-2; alternative Bezeichnung: Polyamid 66; im Folgenden abgekürzt mit "PA66").

PA6 und PA66 nehmen bedingt durch die höhere Dichte an Amid-Gruppen ein Mehrfaches an Wasser auf. Weiterhin liegt das Reaktionsgleichgewicht bei kurzkettigen Polyamiden deutlich weiter auf Seite der Monomere, so dass die Hydrolyse deutlich schneller laufen kann.

Das Recycling dieser Materialien erfordert ein Spalten der Amid-Bindung, welche üblicherweise sauer oder basisch katalysiert erfolgt, um zu definierten Monomerbausteinen zu gelangen.

Erste Ansätze zur chemischen Wiederverwertung von Nylon 66 werden verfolgt, seitdem es diese Materialien gibt.

US 2,872,420 beschreibt ein Verfahren zur Rückgewinnung von Nylon 66 aus Abfällen mit Schwefelsäure. Schwefelsäure hoher Reinheit hat demnach nur einen geringen Einfluss auf das Molekulargewicht von Polyamiden und kann so für eine Aufreinigung durch Auflösen und Ausfällen genutzt werden.

Das Recycling von Nylon 6 ist ein spezieller Fall, da sich das Monomer Caprolactam destillativ aus dem Reaktionsgemisch abtrennen lässt und die Reaktion so auch mit nicht-quantitativem Zusatz von Säure oder Base zu einem hohen Umsatz gebracht werden kann.

Die Behandlung von PA6 und PA66 mit Basen oder mineralischen Säuren bei höheren Temperaturen in Anwesenheit von Wasser führt zu deren Abbau in die Monomerbausteine.

US 2,407,896 A und US 3,069,465 A beschreiben die Hydrolyse von PA 66 mit Schwefelsäure.

WO 97/00846 A1 beschreibt die Hydrolyse von PA66 mit Salpetersäure.

US 2,840,606 A beschreibt die alkalische Hydrolyse von kurzkettigen AABB-Typ Polyamiden. Alkalisch katalysiert bilden sich als Komponenten das Diamin und die Disäure bei Temperaturen größer 160 °C zurück. Die Zugabe aliphatischer Alkohole beschleunigt die Reaktion.

Während die Hydrolyse von PA6 mit den im Stand der Technik beschriebenen Verfahren funktioniert, ist die Aufarbeitung von wässrigen Lösungen dieser Aminosäure schwierig, da die kurzkettigen ω-Aminocarbonsäuren sowohl als Salz als auch als freie Säure gut löslich sind und daher die Reinigung die Destillation großer Wassermengen erfordert.

CN102399363 A beschreibt die Hydrolyse von Nylon-6 durch hydrolytischen Abbau über festen Säuren, die SO₄²⁻-Gruppen aufweisen. Es wird Weg beschrieben, daraus Schmelzkleber herzustellen. Die Reaktion wird nicht zu einem vollständigen Umsatz gebracht.

DE1240087B beschreibt die Aufarbeitung längerkettiger ω-Aminocarbonsäuren wie ω-Aminododecansäure.

Die hydrolytische Wiederaufbereitung von Polyamid 12 ist aufgrund seiner höheren Stabilität technisch anspruchsvoller.

JPS55-108453 A beschreibt den Abbau von Polyamid 12 durch Zusatz von Wasser und Phosphorsäure bei der Extrusion. Es handelt sich aber nur um den Abbau von Molekulargewicht auf einen Wert, der oberhalb des Wertes des Monomers liegt. Dieser Abbau geht demnach nicht bis hin zur Aminolaurinsäure.

DE 693 09 709 T2 beschreibt die Hydrolyse und Oxidation von Polyamiden. Als mögliches Polyamid werden PA6, PA66 und PA12 genannt. Die Reaktion beruht darauf, dass im Hydrolysemedium Nitrosogruppen gelöst sind.

DE 3401415 A beschreibt die Aufarbeitung eines Abfallstromes aus der Herstellung von Laurinlactam. Dieser Abfallstrom besteht aus einer Mischung aus Laurinlactam, Oligo- und Polyamiden sowie weiteren Verunreinigungen. Über säure- bzw. basenkatalysierte Hydrolyse gelingt die Umsetzung zur ω-Aminolaurinsäure.

Es wurde nun festgestellt, dass die Hydrolyse in dem in DE 3401415 A beschriebenen Verfahren durch Laurinlactam als Weichmacher erleichtert wurde. PA-12-Abfallstoffe umfassen Laurinlactam in den meisten Fällen jedoch nicht.

Daneben beschreibt die US 4,170,588 A ein Verfahren zur Hydrolyse von Polylaurinlactam mit Schwefelsäure bei 80 °C. US 2019/0016668 A1 offenbart ein Verfahren zur Co-Synthese von Dicarbonsäuren und ω-Aminocarbonsäuren, ausgehend von Diketonen als Edukten.

Die Aufgabe der vorliegenden Erfindung bestand demnach darin, ein verbessertes Verfahren zu Hydrolyse von Polyamid-12-Abfallstoffen zur Verfügung zu stellen.

Es wurde nun überraschend ein Verfahren gefunden, welches diese Aufgabe löst.

### Kurzbeschreibung der Erfindung

Die vorliegende Erfindung betrifft demnach ein Verfahren zur Hydrolyse von Polylaurinlactam,
wobei Polylaurinlactam mit Schwefelsäure bei einer Temperatur von 125 °C bis 190 °C, bevorzugt 140 °C bis 180 °C, noch bevorzugter 150 °C bis 170 °C, noch mehr bevorzugter 160 bis 165 °C in ω-Aminolaurinsäure gespalten wird, wobei so viel Schwefelsäure eingesetzt wird, dass das Verhältnis der Gewichte von eingesetzter Schwefelsäure zu eingesetztem Polylaurinlactam im Bereich 1 : 0.1 bis 1 : 1 liegt.

### Detaillierte Beschreibung der Erfindung

Es hat sich überraschend gezeigt, dass die Hydrolyse von Polylaurinlactam mit Schwefelsäure gegenüber den im Stand der Technik beschriebenen Verfahren mit einer höheren Ausbeute verläuft.

Das Polylaurinlactam, welches im erfindungsgemäßen Verfahren eingesetzt wird, ist nicht weiter beschränkt. Bevorzugt wird wiederzuverwertendes Material (PA12-Abfall) genutzt.

Das Polylaurinlactam wird insbesondere als Pulver eingesetzt, da damit die Hydrolyse durch die Vergrößerung der Oberfläche beschleunigt werden kann. Geeignetes pulverförmiges PA12 ist beispielsweise in EP 0 911 142 A1 beschrieben. Dazu kann PA12, bevor es im erfindungsgemäßen Verfahren eingesetzt wird, mit dem Fachmann bekannten Verfahren zu Pulver zerrieben werden.

Das erfindungsgemäß eingesetzte PA12 umfasst Wiederholungseinheiten der vorgenannten Struktur **(I),** wobei die mit (*) bezeichnete Bindung einer Wiederholungseinheit an die mit (**) der benachbarten Wiederholungseinheit bindet.

Bevorzugt hat das erfindungsgemäß eingesetzte PA12 dabei eine Molmasse von 1000 bis 10⁶ g/mol, bevorzugter von 3000 bis 200000 g/mol, noch bevorzugter von 15000 bis 150000 g/mol, noch bevorzugter 25000 bis 120000 g/mol, noch mehr bevorzugter 40000 bis 95000 g/mol, noch bevorzugter 80000 g/mol.

Insbesondere eignet sich die vorliegende Erfindung zur Hydrolyse von Polylaurinlactam mit einem geringen Gehalt an Laurinlactam. Bevorzugt wird Polylaurinlactam eingesetzt, was einen Gehalt an Laurinlactam von < 4.9 Gew.-%, bevorzugt < 4.0 Gew.-%, noch bevorzugter < 3.0 Gew.-%, noch mehr bevorzugter < 1.0 Gew.-%, noch viel mehr bevorzugter < 0.01 Gew.-%, jeweils bezogen auf die Summe der Massen von PA12 und Laurinlactam, aufweist.

Insbesondere eignet sich die vorliegende Erfindung zur Hydrolyse von Polylaurinlactam mit einem geringen Gehalt an ω-Aminolaurinsäure. Bevorzugt wird Polylaurinlactam eingesetzt, was einen Gehalt an ω-Aminolaurinsäure von < 4.9 Gew.-%, bevorzugt < 4.0 Gew.-%, noch bevorzugter < 3.0 Gew.-%, noch mehr bevorzugter < 1.0 Gew.-%, noch viel mehr bevorzugter < 0.3 Gew.-%, jeweils bezogen auf die Summe der Massen von PA12 und ω-Aminolaurinsäure, aufweist.

Dies unterscheidet das vorliegende Verfahren insbesondere vom Verfahren in DE 3401415 A, in dem Rückstände aus der Herstellung von Laurinlactam hydrolysiert werden, was zu einem hohen Anteil von Laurinlactam in dem zu hydrolysierenden Edukt führt.

Demgegenüber eignet sich die Hydrolyse mit Schwefelsäure im erfindungsgemäßen Verfahren besonders gut für die Verwendung von zu recyclendem Material, welches sich durch einen geringen Gehalt an Laurinlactam und ω-Aminolaurinsäure auszeichnet.

Die Hydrolyse kann nach dem Fachmann bekannten Verfahren durchgeführt werden.

Die Temperatur im erfindungsgemäßen Verfahren liegt bei 125 °C bis 190 °C, bevorzugt 140 °C bis 180 °C, noch bevorzugter 150 °C bis 170 °C, noch mehr bevorzugter 160 bis 165 °C.

Der Druck im erfindungsgemäßen Verfahren liegt bevorzugt im Bereich 1 bar bis 100 bar, bevorzugt 10 bar bis 60 bar.

Typischerweise wird das zu hydrolysierende Polylaurinlactam in einem Reaktionsgefäß, beispielsweise einem mit Edelmetall wie Gold oder Edelstahl beschichtetem Autoklaven, vorgelegt und mit der Schwefelsäure versetzt.

Die Schwefelsäure ist dabei üblicherweise eine wässrige Lösung, wobei der Gehalt an Schwefelsäure in der wässrigen Lösung nicht weiter beschränkt ist.

Der Gehalt an Schwefelsäure in der wässrigen Lösung liegt bevorzugt im Bereich 10 bis 90 Gew.-%, bevorzugt bei 20 bis 80 Gew.-%, bevorzugter 30 bis 50 Gew-%, noch bevorzugter 35 bis 40 Gew.-%.

Erfindungsgemäß wird so viel Schwefelsäure eingesetzt, dass das Verhältnis der Gewichte von eingesetzter Schwefelsäure zu eingesetztem PA12 im Bereich 1 : 0.1 bis 1 : 1, bevorzugt im Bereich 1 : 0.2 bis 1 : 0.8, bevorzugter im Bereich 1 : 0.3 bis 1 : 0.7, noch bevorzugter im Bereich 1 : 0.4 bis 1 : 0.6, noch mehr bevorzugter im Bereich 1 : 0.45 bis 1 : 0.51 liegt.

Das erfindungsgemäße Verfahren wird bevorzugt durchgeführt, bis sich mindestens 30 Gew.-%, bevorzugter mindestens 50 Gew.-%, noch bevorzugter mindestens 70 Gew.-%, noch mehr bevorzugter mindestens 90 Gew.-%, noch viel mehr bevorzugter mindestens 99 Gew.-% des eingesetzten PA12 umgesetzt haben.

In einer alternativen besonderen Ausführungsform des erfindungsgemäßen Verfahrens wird, nachdem sich 1 - 99 Gew.-%, bevorzugt 30 - 90 Gew.-%, bevorzugter 50 - 70 Gew.-%, des eingesetzten PA12 umgesetzt haben, Alkalihydroxid zugegeben, um die Schwefelsäure zu neutralisieren (d.h. insbesondere, einen pH von 7 in der Umsetzungsmischung einzustellen), und dann weiteres Alkalihydroxid zugegeben, mit dem als Hydrolysierungskatalysator das übrige PA12 umgesetzt wird.

Als Alkalihydroxid wird insbesondere Lithiumhydroxid, Natriumhydroxid oder Kaliumhydroxid verwendet, bevorzugt Kaliumhydroxid.

In dieser alternativen Ausführungsform wird ausgenutzt, dass die Hydrolyse von PA12 mit einem geringen Gehalt von Laurinlactam oder der sehr ähnlichen ω-Aminolaurinsäure mit Schwefelsäure sehr gut funktioniert. Wenn sich das PA12 bis zu einem gewissen Grad zersetzt hat, kann die übrige hydrolytische Spaltung dann in einem Folgeschritt mit Alkalihydroxiden durchgeführt werden.

Dieser Folgeschritt kann dann bevorzugt wie in DE 3401415 A beschrieben durchgeführt werden.

Insbesondere wird so viel Alkalihydroxid im Folgeschritt eingesetzt, dass nach der Neutralisierung der Schwefelsäure so viel Alkalihydroxid übrig bleibt, dass das Verhältnis der Gewichte von nicht umgesetzten Polylaurinlactam zu übrigem Alkalihydroxid im Bereich 1 : 0.1 bis 1 : 1, bevorzugt im Bereich 1 : 0.2 bis 1 : 0.8, bevorzugter im Bereich 1 : 0.3 bis 1 : 0.7, noch bevorzugter im Bereich 1 : 0.4 bis 1 : 0.6, noch mehr bevorzugter im Bereich 1 : 0.45 bis 1 : 0.51 liegt.

Die Temperatur im Folgeschritt liegt bevorzugt im Bereich 160 °C bis 280 °C, bevorzugt 180 °C bis 250 °C, bevorzugter 200 °C bis 230 °C.

Der Druck im Folgeschritt liegt bevorzugt im Bereich 1 bar bis 100 bar, bevorzugt 10 bar bis 60 bar. Im Folgeschritt wird das Alkalihydroxid bevorzugt als wässrige Lösung, noch bevorzugter mit einer Konzentration von 1 bis 10 Gew.-% an Alkalihydroxid eingesetzt.

Wird der Folgeschritt durchgeführt, kann die übrige Aufarbeitung der gespaltenen PA12 wie in DE 3401415 A beschrieben durchgeführt werden.

Die folgenden Beispiele illustrieren die Erfindung.

### Beispiele

Die Depolymerisierung von PA12 mit einem Gehalt von Laurinlactam und ω-Aminolaurinsäure von jeweils < 1 % wurde mit verschiedenen Katalysatoren und Temperaturbedingungen untersucht.

Es wurden die folgenden Katalysatoren ausprobiert: KOH, H₂SO₄, H₃PO₄.

Die Umsetzungsrate wurde mit ¹H-NMR in CDCl₂ + Trifluoessigsäureanhydrid ermittelt.

Die folgenden Versuche wurden durchgeführt:

### 1. Vergleichsversuch V1: H₃PO₄, 320 °C

6.66 mg PA12 Pulver wurden in einen goldbeschichteten Autoklaven gegeben. Danach wurden 6.8 mg H₃PO₄ (85 %) und 6.30 mg Wasser zugegeben und der Autoklav versiegelt. Der Autoklav wurde in einem Ofen auf 320 °C erhitzt und die Reaktionsmischung für 4 Stunden bei dieser Temperatur gehalten. Danach wurde die Mischung mit einer Rate von 100 K/min auf Raumtemperatur abgekühlt. Die Reaktionsmischung wurde aus dem Reaktor entnommen und durch Zugabe von 1 g Wasser und einer stöchiometrischen Menge an KOH neutralisiert. Die Mischung wurde bei 80 °C und 200 mbar getrocknet. Die Umsetzungsrate des PA12 betrug 96 %.

### 2. Vergleichsversuch V2: H₃PO₄, 300 °C

5.9 mg PA12 Pulver wurden in einen goldbeschichteten Autoklaven gegeben. Danach wurden 4.25 mg H₃PO₄ (85 %) und 5.23 mg Wasser zugegeben und der Autoklav versiegelt. Der Autoklav wurde in einem Ofen auf 300 °C erhitzt und die Reaktionsmischung für 4 Stunden bei dieser Temperatur gehalten. Danach wurde die Mischung mit einer Rate von 100 K/min auf Raumtemperatur abgekühlt. Die Reaktionsmischung wurde aus dem Reaktor entnommen und durch Zugabe von 1 g Wasser und einer stöchiometrischen Menge an KOH neutralisiert. Die Mischung wurde bei 80 °C und 200 mbar getrocknet. Die Umsetzungsrate des PA12 betrug 85 %.

### 3. Vergleichsversuch V3: H₃PO₄, 250 °C

7.17 mg PA12 Pulver wurden in einen goldbeschichteten Autoklaven gegeben. Danach wurden 4.98 mg H₃PO₄ (85 %) und 6.23 mg Wasser zugegeben und der Autoklav versiegelt. Der Autoklav wurde in einem Ofen auf 250 °C erhitzt und die Reaktionsmischung für 4 Stunden bei dieser Temperatur gehalten. Danach wurde die Mischung mit einer Rate von 100 K/min auf Raumtemperatur abgekühlt. Die Reaktionsmischung wurde aus dem Reaktor entnommen und durch Zugabe von 1 g Wasser und einer stöchiometrischen Menge an KOH neutralisiert. Die Mischung wurde bei 80 °C und 200 mbar getrocknet. Die Umsetzungsrate des PA12 betrug 88 %.

### 4. Vergleichsversuch V4: H₃PO₄, 220 °C

6.95 mg PA12 Pulver wurden in einen goldbeschichteten Autoklaven gegeben. Danach wurden 4.6 mg H₃PO₄ (85 %) und 6.30 mg Wasser zugegeben und der Autoklav versiegelt. Der Autoklav wurde in einem Ofen auf 220 °C erhitzt und die Reaktionsmischung für 4 Stunden bei dieser Temperatur gehalten. Danach wurde die Mischung mit einer Rate von 100 K/min auf Raumtemperatur abgekühlt. Die Reaktionsmischung wurde aus dem Reaktor entnommen und durch Zugabe von 1 g Wasser und einer stöchiometrischen Menge an KOH neutralisiert. Die Mischung wurde bei 80 °C und 200 mbar getrocknet. Die Umsetzungsrate des PA12 betrug 87 %.

### 5. Vergleichsversuch V5: H₃PO₄, 200 °C

7.27 mg PA12 Pulver wurden in einen goldbeschichteten Autoklaven gegeben. Danach wurden 5.43 mg H₃PO₄ (85 %) und 6.20 mg Wasser zugegeben und der Autoklav versiegelt. Der Autoklav wurde in einem Ofen auf 200 °C erhitzt und die Reaktionsmischung für 4 Stunden bei dieser Temperatur gehalten. Danach wurde die Mischung mit einer Rate von 100 K/min auf Raumtemperatur abgekühlt. Die Reaktionsmischung wurde aus dem Reaktor entnommen und durch Zugabe von 1 g Wasser und einer stöchiometrischen Menge an KOH neutralisiert. Die Mischung wurde bei 80 °C und 200 mbar getrocknet. Die Umsetzungsrate des PA12 betrug 100 %.

### 6. Erfinderisches Beispiel E1: H₂SO₄, 180 °C

6.5 mg PA12 Pulver wurden in einen goldbeschichteten Autoklaven gegeben. Danach wurden 8.62 mg H₂SO₄ (35 %) zugegeben und der Autoklav versiegelt. Der Autoklav wurde in einem Ofen auf 180 °C erhitzt und die Reaktionsmischung für 4 Stunden bei dieser Temperatur gehalten. Danach wurde die Mischung mit einer Rate von 100 K/min auf Raumtemperatur abgekühlt. Die Reaktionsmischung wurde aus dem Reaktor entnommen und durch Zugabe von 1 g Wasser und einer stöchiometrischen Menge an KOH neutralisiert. Die Mischung wurde bei 80 °C und 200 mbar getrocknet. Die Umsetzungsrate des PA12 betrug 100 %.

### 7. Erfinderisches Beispiel E2: H₂SO₄, 160 °C

6.61 mg PA12 Pulver wurden in einen goldbeschichteten Autoklaven gegeben. Danach wurden 8.63 mg H₂SO₄ (35 %) zugegeben und der Autoklav versiegelt. Der Autoklav wurde in einem Ofen auf 160 °C erhitzt und die Reaktionsmischung für 4 Stunden bei dieser Temperatur gehalten. Danach wurde die Mischung mit einer Rate von 100 K/min auf Raumtemperatur abgekühlt. Die Reaktionsmischung wurde aus dem Reaktor entnommen und durch Zugabe von 1 g Wasser und einer stöchiometrischen Menge an KOH neutralisiert. Die Mischung wurde bei 80 °C und 200 mbar getrocknet. Die Umsetzungsrate des PA12 betrug 98 %.

### 8. Vergleichsversuch V6 (entsprechend DE 3407415 A1): KOH, 320 °C

30.0 g PA12 Pulver wurden in einen nickelbeschichteten Autoklaven gegeben. Danach wurden 18.95 g KOH-Lösung (50 %) in Wasser zugegeben und der Autoklav versiegelt. Der Autoklav wurde auf 28 bar mit Stickstoff gedrückt und auf 320 °C erhitzt. Die Reaktionsmischung wurde für 4 Stunden bei dieser Temperatur gehalten. Danach wurde die Mischung mit einer Rate von 100 K/min auf Raumtemperatur abgekühlt und der Druck abgelassen. Die Reaktionsmischung wurde aus dem Reaktor entnommen, in Wasser gelöst und durch Zugabe von 13 g Schwefelsäure neutralisiert. Die Mischung wurde bei 70 °C und 100 mbar getrocknet. Die Umsetzungsrate des PA12 betrug 94 %.

### 9. Vergleichsversuch V7 (entsprechend DE 3407415 A1): KOH, 300 °C

7.42 mg PA12 Pulver wurden in einen goldbeschichteten Autoklaven gegeben. Danach wurden 4.03 mg KOH-Lösung (50 %) in Wasser zugegeben und der Autoklav versiegelt. Der Autoklav wurde in einem Ofen auf 300 °C erhitzt. Die Reaktionsmischung wurde für 4 Stunden bei dieser Temperatur gehalten und dann mit einer Rate von 100 K/min auf Raumtemperatur abgekühlt. Danach wurde die Mischung auf Raumtemperatur abgekühlt. Die Reaktionsmischung wurde aus dem Reaktor entnommen und durch Zugabe von 1 g Wasser und stöchiometrischer Menge an Phosphorsäure neutralisiert. Die Mischung wurde bei 70 °C und 100 mbar getrocknet. Die Umsetzungsrate des PA12 betrug 78 %.

### 10. Vergleichsversuch V8 (entsprechend DE 3407415 A1): KOH, 280 °C

7.17 mg PA12 Pulver wurden in einen goldbeschichteten Autoklaven gegeben. Danach wurden 4.42 mg KOH-Lösung (50 %) in Wasser zugegeben und der Autoklav versiegelt. Der Autoklav wurde in einem Ofen auf 280 °C erhitzt. Die Reaktionsmischung wurde für 4 Stunden bei dieser Temperatur gehalten und dann mit einer Rate von 100 K/min auf Raumtemperatur abgekühlt. Danach wurde die Mischung auf Raumtemperatur abgekühlt. Die Reaktionsmischung wurde aus dem Reaktor entnommen und durch Zugabe von 1 g Wasser und stöchiometrischer Menge an Phosphorsäure neutralisiert. Die Mischung wurde bei 70 °C und 100 mbar getrocknet. Die Umsetzungsrate des PA12 betrug 78 %.

### 11. Vergleichsversuch V9 (entsprechend DE 3407415 A1): KOH, 280 °C

6.80 mg PA12 Pulver wurden in einen goldbeschichteten Autoklaven gegeben. Danach wurden 0.95 mg KOH-Lösung (50 %) in Wasser zugegeben und der Autoklav versiegelt. Der Autoklav wurde in einem Ofen auf 320 °C erhitzt. Die Reaktionsmischung wurde für 4 Stunden bei dieser Temperatur gehalten und dann mit einer Rate von 100 K/min auf Raumtemperatur abgekühlt. Danach wurde die Mischung auf Raumtemperatur abgekühlt. Die Reaktionsmischung wurde aus dem Reaktor entnommen und durch Zugabe von 1 g Wasser und stöchiometrischer Menge an Phosphorsäure neutralisiert. Die Mischung wurde bei 70 °C und 100 mbar getrocknet. Die Umsetzungsrate des PA12 betrug 2 %.

### 12. Vergleichsversuch V10: ohne Katalysator, 320 °C

7.62 mg PA12 Pulver wurden in einen nickelbeschichteten Autoklaven gegeben. Danach wurden 3.91 mg Wasser zugegeben und der Autoklav versiegelt. Der Autoklav wurde in einem Ofen auf 320 °C erhitzt. Die Reaktionsmischung wurde für 4 Stunden bei dieser Temperatur gehalten und dann mit einer Rate von 100 K/min auf Raumtemperatur abgekühlt. Die Reaktionsmischung wurde aus dem Reaktor entnommen und bei 80 °C und 200 mbar getrocknet. Die Umsetzungsrate des PA12 betrug 6 %.

Die folgende Tabelle 1 fasst die Ergebnisse der Versuche und Vergleichsversuche zusammen.

**Tabelle 1**

| Versuch | Menge PA-12 in mg | Katalysator | Menge Katalysator bezogen auf Menge PA-12 in Gew.-% | Temperatur | Ausbeute |
|---|---|---|---|---|---|
| **V1** | 6.66 | H₃PO₄ | 86.7 | 320 °C | 96 % |
| **V2** | 5.9 | H₃PO₄ | 61.2 | 300 °C | 85 % |
| **V3** | 7.17 | H₃PO₄ | 59.0 | 250 °C | 88 % |
| **V4** | 6.95 | H₃PO₄ | 56.3 | 220 °C | 87 % |
| **V5** | 7.27 | H₃PO₄ | 63.5 | 200 °C | 100 % |
| **E1** | 6.5 | H₂SO₄ | 46.4 | 180 °C | 100 % |
| **E2** | 6.61 | H₂SO₄ | 45.7 | 160 °C | 98 % |
| **V6** | 30000.0 | KOH | 31.6 | 320 °C | 94 % |
| **V7** | 7.42 | KOH | 27.1 | 300 °C | 78 % |
| **V8** | 7.17 | KOH | 30.8 | 280 °C | 78 % |
| **V9** | 6.8 | KOH | 6.99 | 320 °C | 2% |
| **V10** | 7.62 | - | - | 320 °C | 6% |

Aus den Ergebnissen ist ersichtlich, dass die Zugabe von H₂SO₄ bei besonders niedrigen Temperaturen, d.h. schon bei 190 °C und 160 °C, eine fast quantitative Umsetzung erbringt.

### 13. Temperaturabhängigkeit der Hydrolyse von PA12 mit Schwefelsäure

Um die Temperaturabhängigkeit der Hydrolyse von PA12 mit Schwefelsäure zu untersuchen, wurde die folgende Versuchsreihe durchgeführt:
PA12 Pulver und H₂SO₄ (35 %) wurden in einem goldbeschichteten Autoklaven in der jeweils in der folgenden Tabelle 2 angegebenen Menge zugegeben. Danach wurde der Autoklav versiegelt. Der Autoklav wurde in einem Ofen auf die in der folgenden Tabelle 2 angegebene jeweilige Temperatur **T** erhitzt (Heizrate 5 K/min) und die Reaktionsmischung für 4 Stunden bei dieser Temperatur gehalten. Danach wurde die Mischung mit einer Rate von 5 K/min auf Raumtemperatur abgekühlt. Danach wurde der Restgehalt an PA12 bestimmt.

**Tabelle 2**

| Versuch | Menge PA-12 in mg | Zugegebene Menge an 35 % H₂SO₄ in mg | Temperatur T | Restgehalt an PA12 |
|---|---|---|---|---|
| **V11** | 5.33 | 5.08 | 80 °C | 90.5 % |
| **E3** | 5.47 | 5.33 | 125 °C | 7.3 % |
| **E4** | 5.7 | 4.99 | 158 °C | 6.3 % |
| **E5** | 6.22 | 5.32 | 190 °C | 5.3 % |
| **V12** | 6.3 | 5.1 | 230 °C | 10.5 % |
| **V13** | 6.74 | 12.68 | 80 °C | 7.2 % |
| **E6** | 5.60 | 12.07 | 125 °C | < 0.1 % |
| **E7** | 5.03 | 13.04 | 158 °C | 1.3 % |
| **E8** | 6.78 | 12.07 | 190 °C | 0.7 % |
| **V14** | 5.74 | 13.23 | 230 °C | 3.2 % |

Anhand der in Tabelle 2 gezeigten Versuchsergebnisse ist ersichtlich, dass die Hydrolyse von PA12 mit Schwefelsäure im Temperaturbereich von 125 °C bis 190 °C **(E3** bis **E5; E6** bis **E8)** überraschend effizient läuft, wobei **E3** bis **E5** nicht erfindungsgemäß sind.

Im Vergleich dazu ist die Hydrolyse bei einer Temperatur von 80 °C (Vergleichsversuche **V11, V13)** die im Stand der Technik erfolgt (US 4,170,588 A), nicht ergiebig. Die Vergleichsversuche **V12** und **V14** belegen außerdem, dass bei einer Temperatur von 230 °C der Restgehalt an PA12 wieder ansteigt, die Effizienz der Umsetzung somit wieder abnimmt.

## Patentansprüche

1. Verfahren zur Hydrolyse von Polylaurinlactam, wobei Polylaurinlactam mit Schwefelsäure bei einer Temperatur von 125 °C bis 190 °C in ω-Aminolaurinsäure gespalten wird, wobei so viel Schwefelsäure eingesetzt wird, dass das Verhältnis der Gewichte von eingesetzter Schwefelsäure zu eingesetztem Polylaurinlactam im Bereich 1 : 0.1 bis 1 : 1 liegt.

2. Verfahren nach Anspruch 1, wobei das Polylaurinlactam eine Molmasse von 1000 bis 10⁶ g/mol aufweist.

3. Verfahren nach Anspruch 1 oder 2, wobei Polylaurinlactam eingesetzt wird, was einen Gehalt an Laurinlactam von < 4.9 Gew.-% aufweist.

4. Verfahren nach einem der Ansprüche 1 bis 3, wobei Polylaurinlactam eingesetzt wird, was einen Gehalt an ω-Aminolaurinsäure von < 4.9 Gew.-% aufweist.

5. Verfahren nach einem der Ansprüche 1 bis 4, wobei der Druck bei 1 bar bis 100 bar liegt.

6. Verfahren nach einem der Ansprüche 1 bis 5, wobei, nachdem sich 1 - 99 Gew.-% des eingesetzten Polylaurinlactam umgesetzt haben, Alkalihydroxid zugegeben wird, um die Schwefelsäure zu neutralisieren, und dann weiteres Alkalihydroxid zugegeben, mit dem das übrige Polylaurinlactam umgesetzt wird.

7. Verfahren nach Anspruch 6, wobei so viel Alkalihydroxid im Folgeschritt eingesetzt wird, dass nach der Neutralisierung der Schwefelsäure so viel Alkalihydroxid übrig bleibt, dass das Verhältnis der Gewichte von nicht umgesetzten Polylaurinlactam zu übrigem Alkalihydroxid im Bereich 1 : 0.1 bis 1 : 1 liegt.

8. Verfahren nach Anspruch 6 oder 7, wobei die Temperatur im Folgeschritt im Bereich 160 °C bis 280 °C liegt.

9. Verfahren nach einem der Ansprüche 6 bis 8, wobei der Druck im Folgeschritt im Bereich 1 bis 100 bar liegt.

## Claims

1. Process for the hydrolysis of polylaurolactam, wherein polylaurolactam is cleaved with sulfuric acid at a temperature of from 125°C to 190°C to give ω-aminolauric acid, wherein sulfuric acid is used in such an amount that the ratio of the weights of sulfuric acid used to polylaurolactam used is in the range 1 : 0.1 to 1 : 1.

2. Process according to Claim 1, wherein the polylaurolactam has a molar mass of 1000 to 10⁶ g/mol.

3. Process according to Claim 1 or 2, wherein polylaurolactam is used which has a content of laurolactam of < 4.9% by weight.

4. Process according to any of Claims 1 to 3, wherein polylaurolactam is used which has a content of ω-aminolauric acid of < 4.9% by weight.

5. Process according to any of Claims 1 to 4, wherein the pressure is 1 bar to 100 bar.

6. Process according to any of Claims 1 to 5, wherein, after 1-99% by weight of the polylaurolactam used has reacted, alkali metal hydroxide is added in order to neutralize the sulfuric acid, and then further alkali metal hydroxide is added with which the remaining polylaurolactam is reacted.

7. Process according to Claim 6, wherein alkali metal hydroxide is used in such an amount in the subsequent step that, after the neutralization of the sulfuric acid, alkali metal hydroxide remains in such an amount that the ratio of the weights of unconverted polylaurolactam to remaining alkali metal hydroxide is in the range 1 : 0.1 to 1 : 1.

8. Process according to Claim 6 or 7, wherein the temperature in the subsequent step is in the range 160°C to 280°C.

9. Process according to any of Claims 6 to 8, wherein the pressure in the subsequent step is in the range 1 bar to 100 bar.

## Revendications

1. Procédé d'hydrolyse de polylauryllactame, dans lequel le polylauryllactame est dissocié en acide ω-aminolaurique à l'aide d'acide sulfurique à une température de 125°C à 190°C, la quantité d'acide sulfurique utilisée étant telle que le rapport du poids d'acide sulfurique utilisé au poids de polylauryllactame utilisé se situe dans la plage de 1:0,1 à 1:1.

2. Procédé selon la revendication 1, dans lequel le polylauryllactame présente une masse molaire de 1000 à 10⁶ g/mole.

3. Procédé selon la revendication 1 ou 2, dans lequel le polylauryllactame utilisé présente une teneur en lauryllactame < 4,9% en poids.

4. Procédé selon l'une des revendications 1 à 3, dans lequel le polylauryllactame utilisé présente une teneur en acide ω-aminolaurique < 4,9% en poids.

5. Procédé selon l'une des revendications 1 à 4, dans lequel la pression se situe à 1 bar jusqu'à 100 bars.

6. Procédé selon l'une des revendications 1 à 5, dans lequel, après la transformation de 1 - 99% en poids du polylauryllactame, de l'hydroxyde de métal alcalin est ajouté afin de neutraliser l'acide sulfurique, puis de l'hydroxyde de métal alcalin supplémentaire est ajouté, avec lequel le polylauryllactame qui reste est transformé.

7. Procédé selon la revendication 6, dans lequel la quantité d'hydroxyde de métal alcalin ajoutée dans l'étape ultérieure est telle qu'il reste, après la neutralisation de l'acide sulfurique, une quantité d'hydroxyde de métal alcalin telle que le rapport du poids de polylauryllactame non transformé au poids d'hydroxyde de métal alcalin qui reste se situe dans la plage de 1:0,1 à 1:1.

8. Procédé selon la revendication 6 ou 7, dans lequel la température dans l'étape ultérieure se situe dans une plage de 160°C à 280°C.

9. Procédé selon l'une des revendications 6 à 8, dans lequel la pression dans l'étape ultérieure se situe dans la plage de 1 à 100 bars.
